# EUROPÄISCHE PATENTSCHRIFT

(11) **EP 3 353 299 B1**
(45) Veröffentlichungstag und Bekanntmachung des Hinweises auf die Patenterteilung: **04.03.2020**
(21) Anmeldenummer: 16766834.2
(22) Anmeldetag: 09.08.2016
(51) Int. Cl.: C12N 15/113, C12N 15/11

(54) **VERFAHREN UND SUBSTANZEN ZUR GERICHTETEN RNA-EDITIERUNG**
METHODS AND SUBSTANCES FOR DIRECTED RNA EDITING
PROCÉDÉS ET SUBSTANCES POUR L'ÉDITION DIRIGÉE D'ARN

(30) Priorität: 26.09.2015 DE 102015012522
(43) Veröffentlichungstag der Anmeldung: 01.08.2018
(73) Patentinhaber: Eberhard Karls Universität Tübingen, 72074 Tübingen (DE)
(72) Erfinder: STAFFORST, Thorsten, 72074 Tübingen (DE); WETTENGEL, Jacqueline, 72074 Tübingen (DE); VOGEL, Paul, 72074 Tübingen (DE)
(74) Vertreter: Graf von Stosch Patentanwaltsgesellschaft mbH
(86) Internationale Anmeldenummer: PCT/DE2016/000309
(87) Internationale Veröffentlichungsnummer: WO 2017/050306

(56) Entgegenhaltungen:
- M.F. SCHNEIDER ET AL.: "Optimal guideRNAs for re-directing deaminase activity of hADAR1 and hADAR2 in trans", NUCLEIC ACIDS RESEARCH, Bd. 42, Nr. 10, 17. April 2014 (2014-04-17), Seiten e87-e87, XP055260764, GB ISSN: 0305-1048, DOI: 10.1093/nar/gku272 -& M.F. Schneider ET AL.: "Optimal guideRNAs for re-directing Deaminase activity of hADAR1 and hADAR2 in trans", , 1. Januar 2014 (2014-01-01), XP055329590, Gefunden im Internet: URL:http://nar.oxfordjournals.org/content/ suppl/2014/04/15/gku272.DC1/nar-03496-met- g-2013-File007.pdf [gefunden am 2016-12-15]
- STAFFORST THORSTEN ET AL.: "An RNA-deaminase conjugate selectively repairs point mutations", ANGEWANDTE CHEMIE (INTERNATIONAL ED. IN ENGLISH), Bd. 51, Nr. 44, 29. Oktober 2012 (2012-10-29), Seiten 11166-11169, XP002765253, ISSN: 1521-3773
- J. WETTENGEL ET AL.: "Harnessing human ADAR2 for RNA repair - recoding a PINK1 mutation rescues mitophagy", NUCLEIC ACIDS RESEARCH, 7. Oktober 2016 (2016-10-07), Seite gkw911, XP55329586, GB ISSN: 0305-1048, DOI: 10.1093/nar/gkw911 -& J. Wettengel et al.: "SupportingInformation HarnessinghumanADAR2forRNArepair-Recodinga PINK1mutationrescuesmitophagy", , 1. Januar 2016 (2016-01-01), XP55329589, Gefunden im Internet: URL:http://nar.oxfordjournals.org/content/ suppl/2016/10/07/gkw911.DC1/nar-02482-a-20 16-File006.pdf [gefunden am 2016-12-15]
- VOGEL P.; STAFFORST T.: "Site-directed RNA editing with antagomir deaminases - a tool to study protein and RNA function.", CHEMMEDCHEM, Bd. 9, 2014, Seiten 2021-2025, XP002765254, in der Anmeldung erwähnt
- VOGEL P.; SCHNEIDER M.F.; WETTENGEL J.; STAFFORST T.: "Improving site-directed RNA editing in vitro and in cell culture by chemical modification ofthe guideRNA", ANGEWANDTE CHEMIE, Bd. 53, 2014, Seiten 6267-6271, XP002765255, in der Anmeldung erwähnt

## Beschreibung

Die vorliegende Erfindung betrifft ein Verfahren und Substanzen zur gezielten Veränderung genetischer Information auf der RNA-Ebene.

Das Nukleosid Adenosin kann enzymatisch desaminiert werden. Dabei entsteht Inosin, welches bei einer Translation wie Guanosin gelesen wird. Diese Nukleosid-Änderung wird als A-nach-I-Editierung bezeichnet und kann dazu verwendet werden, einzelne Punktmutationen in RNA einzufügen, um daraus resultierende Proteine gezielt zu verändern.

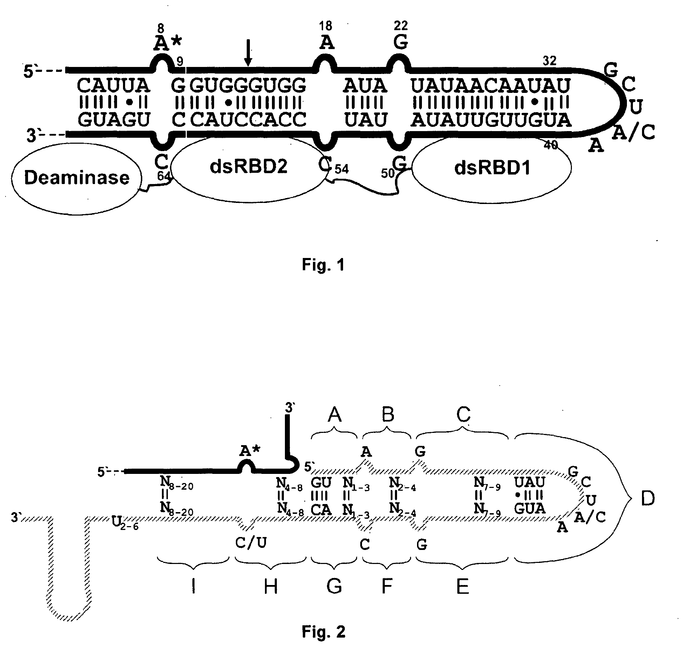

Von den natürlich vorkommenden Enzymen hADARs (human adenosine deaminase acting on RNA) ist bekannt, dass sie Adenosin desaminieren können und dabei hoch promiskuitiv sind **(5).** So kann das Enzym hADAR2 tausende verschiedene Substrate erkennen und editieren, wobei als Substrat fast jede Doppelstrang-RNA mit einer Länge von mehr als 30 Basenpaaren dienen kann. Dennoch werden nur einige wenige Substrate hochselektiv und effizient editiert (7). Der Grund dafür scheint die Erkennung spezieller Sequenzen und Sekundärstrukturen durch die dsRBD (dsRNA binding domains) zu sein. ADAR2 hat zwei dsRBD (1&2) im N-Terminus. An Modelsubstraten, wie dem GluR2-Transkript, konnte gezeigt werden, dass die dsRBD1&2 an jeweils verschiedene Bereiche im GluR2-Transkript in definierten Modi binden **(8,** **Fig. 1****).** Am C-terminalen Ende von hADAR2 ist die Deaminase-Domäne platziert.

Aus dem Stand der Technik ist eine Methode bekannt, wie man ihre Enzymaktivität hoch-spezifisch und RNA-abhängig an neue Substrate lenken kann **(1, 2, 4, 6, 9).** Dabei werden die dsRBD-Proteindomänen durch ein so genanntes SNAP-tag ersetzt, an das eine speziell konstruierte guide-RNA kovalent gekoppelt ist. Durch diese speziell konstruierte anhängende guide-RNA wird das o.g. SNAP-tag-Konstrukt aktiviert.

Bei einem anderen bekannten Verfahren wird dem natürlichen hADAR2 ein In-peptid angefügt und der guide-RNA ein BoxB-motif, um die Bindung zu ermöglichen **(3).**

Ein großer Nachteil der aus dem Stand der Technik bekannten Verfahren besteht darin, dass sie nicht das natürlich vorkommende ADAR2 Enzym nutzen, sondern die mit dem SNAP-tag bzw. In-peptid modifizierte Enzymvariante. Ein weiterer Nachteil besteht darin, dass die guide-RNA chemisch veränderte Nukleotide, wie z.B. Benzylguanin (BG), enthält, die nicht genetisch kodierbar sind. Die guide-RNA muss deswegen bei diesen Verfahren zuerst *in vitro* hergestellt und erst dann in die Zelle transfiziert werden.

Die Aufgabe der vorliegenden Erfindung ist es, ein Verfahren und Substanzen bereit zu stellen, mit denen es möglich ist, Punktmutationen in Genprodukte (RNA) effizient und reversibel einzubringen, ohne das kodierende Gen zu verändern.

Diese Aufgabe wird durch die Bereitstellung von speziell konstruierten, genetisch kodierbaren guide-RNAs gelöst, die in der Lage sind, endogene Editierungsenzyme zu rekrutieren, um gezielt Punktmutationen in ausgesuchten RNAs einzubringen.

Im Einzelnen enthält die erfindungsgemäße guide-RNA mindestens folgende aneinandergekoppelte Nukleotid-Segmente, vom 5'-Ende aufgelistet **(****Fig. 2****):**
- Segment A: Nukleotid-Sequenz mit einer Länge von drei bis fünf Basen, wobei in der Position 1 immer ein Guanosin und in der Position 2 immer ein Uridin steht;
- Segment B: Nukleotid-Sequenz mit einer Länge von drei bis fünf Basen, wobei in der Position 1 immer ein Adenosin steht;
- Segment C: Nukleotid-Sequenz mit einer Länge von acht bis zehn Basen, wobei in der Position 1 immer ein Guanosin steht;
- Segment D: Nukleotid-Sequenz UAUGCUAAAUG oder UAUGCUCAAUG;
- Segment E: Nukleotid-Sequenz mit einer Länge von acht bis zehn Basen, wobei in der letzten Position immer ein Guanosin steht;
- Segment F: Nukleotid-Sequenz mit einer Länge von drei bis fünf Basen, wobei in der letzten Position immer ein Cytosin steht;
- Segment G: Nukleotid-Sequenz mit einer Länge von drei bis fünf Basen, wobei in der vorletzten Position immer ein Adenosin und in der letzten Position ein Cytosin steht;
- Segment H: Nukleotid-Sequenz mit einer Länge von fünf bis neun Basen, wobei in der letzten Position immer ein Cytosin oder ein Uridin steht;
- Segment I: Nukleotid-Sequenz mit einer Länge von acht bis zwanzig Basen,

Dabei paaren sich einzelne Nukleotide der Segmente A und G, B und F bzw. C und E zu einer Doppelhelix und die Nukleotide des Segments D bilden eine Haarnadelstruktur, wobei Segmente H und I so konstruiert sind, dass sie mit der zu editierenden mRNA paaren **(****Fig. 2****).**

Das Segment A beträgt insbesondere die Länge von vier Nukleotiden und weist die Nukleotid-Sequenz GUGG auf.

Das Segment B beträgt insbesondere die Länge von vier Nukleotiden und weist die Nukleotid-Sequenz AAUA auf.

Das Segment C beträgt insbesondere die Länge von neun Nukleotiden und weist die Nukleotid-Sequenz GUAUAACAA auf.

Das Segment E beträgt insbesondere die Länge von neuen Nukleotiden und weist die Nukleotid-Sequenz UUGUUAUAG auf.

Das Segment F beträgt insbesondere die Länge von vier Nukleotiden und weist die Nukleotid-Sequenz UAUC auf.

Das Segment G beträgt insbesondere die Länge von vier Nukleotiden und weist die Nukleotid-Sequenz CCAC auf.

Die Segmente H und I sind so konstruiert, dass sie mit der Ziel-mRNA paaren und die zu editierende Zielbase in ein A:C Fehlpaar platzieren.

In einer speziellen Ausführung der Erfindung wird der guide-RNA am 3'-Ende zur Stabilisierung zusätzlich eine Haarnadelstruktur, z.B. ein BoxB-Motiv, angehängt.

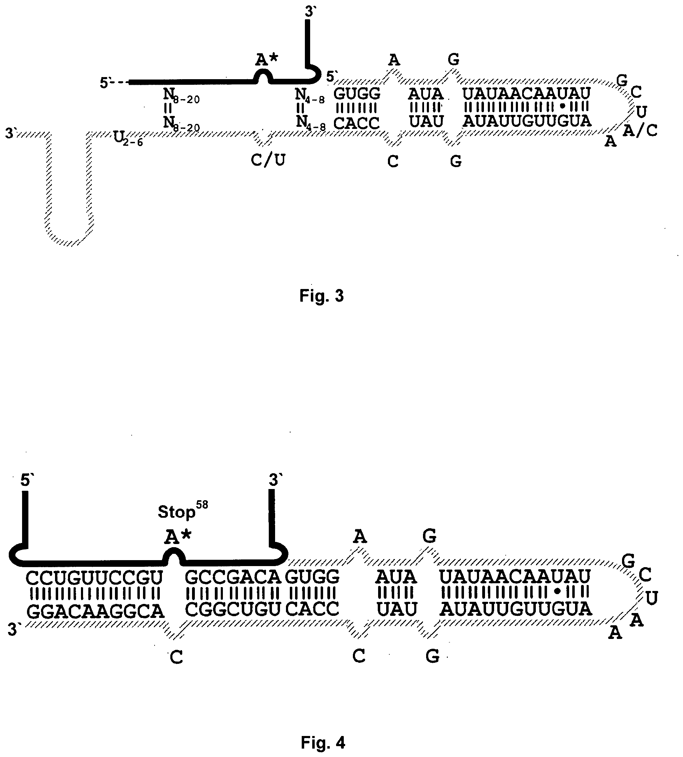

Die Struktur der besonders bevorzugten Ausführung der erfindungsgemäßen guide-RNA ist in der **Fig. 3** dargestellt.

Bei den Editierungsenzymen geht es vor allem um hADAR-Enzyme, insbesondere um die Enzyme hADAR2 oder hADAR1. Da beide Enzyme unterschiedliche Substrate bedienen und unterschiedlich in Körpergeweben expimiert sind, können erfindungsgemäß über die Auswahl der Editierungsenzyme bestimmte Gewebe bzw. Zellen gezielt angesteuert werden.

Mittels eines auf diese Weise erzielten Nukleotid-Austausches können Reparaturen einzelner Punktmutationen, wie beispielsweise solche, die zu vorzeitigen Stopp-Signalen führen, vorgenommen werden. Dabei kann der Nukleotid-Austausch an einem oder an mehreren Stellen der Ziel-RNAs erzielt werden, sodass auch multigenetische Krankheiten mit dem erfindungsgemäßen Verfahren behandelt werden können.

Erfindungsgemäß können therapierelevante guide-RNAs entweder über einzelne Gaben an die Patienten gezielt, z.B. in betroffenen Organen oder Zellen verabreicht werden, oder sie können über eine Installation in den Zellen bzw. Organen des Patienten dauerhaft exprimiert werden. Die Übertragung der therapeutischen guide-RNA kann dabei z.B. viral, als stabilisierte mRNA oder genetisch codiert erfolgen. Die erwünschten Punktmutationen können dabei gewebespezifisch, induzierbar oder reversibel geschaltet werden. Ebenfalls kann das Ausmaß der Editierung nach Bedarf eingestellt und abhängig von konkreten Gegebenheiten und Therapiezielen angepasst werden, so dass eine Editierung zu einem Grad zwischen 0 und 100% erzielt werden kann.

Da die ADAR-Deaminasen in Säugertieren insbesondere in Neuronen stark expimiert werden, kann das erfindungsgemäße Verfahren zur Behandlung von neuronalen Erkrankungen eingesetzt werden, die beispielsweise durch einzelne Punktmutationen hervorgerufen werden. Dabei könnte die intrinsisch vorhandenen ADARs durch eine einfache Gabe oder eine ektopische Expression von kleinen synthetischen guide-RNAs zu einer korrigierenden RNA-Editierung rekrutiert werden.

Ein großer Vorteil des erfindungsgemäßen Verfahrens besteht darin, dass dabei endogene, d.h. zelleigene Editierungsenzyme verwendet werden, um gezielte Punktmutationen in die RNA einzubringen. Nur die kurze guide-RNA, die erfindungsgemäß zur Rekrutierung endogener Editierungsenzyme verwendet wird, muss für jede spezielle Fragestellung künstlich hergestellt und ektopisch exprimiert werden. Dadurch wird die gezielte Veränderung von Proteinen auf Translationsebene besonders einfach, so dass eine Korrektur krankheitsverursachender Punktmutationen ermöglicht wird. Im Vergleich zum Stand der Technik ist es beim erfindungsgemäßen Verfahren nicht notwendig, ein zusätzliches, körperfremdes Protein zu exprimieren, was insbesondere für die medizinische Anwendung von großem Vorteil ist. Auch die Effizienz ist höher und die unerwünschten Nebeneffekte geringer, weil nur ein statt zwei oder mehr Konstrukte an die Zielgewebe übertragen werden müssen.

Das Verfahren ist wegen der Kodierbarkeit der verwendeten Konstrukte besonders einfach und günstig, denn die Synthese chemisch stabilisierter guide-RNAs entfällt: die guide-RNA wird auf einem Plasmid kodiert, in die Zelle transfiziert und von der Zelle selbst gebildet.

Weitere Vorteile, Merkmale und Anwendungsmöglichkeiten der Erfindung werden nachstehend anhand der unten beschriebenen Ausführungsbeispiele mit Bezug auf die Figuren beschrieben.

In den Figuren stellt die gestrichelte Linie die konstruierte erfindungsgemäße guide-RNA, die durchgezogene Linie die zu editierende Target-mRNA dar; das Sternchen markiert das zu editierende Nukleotid in der Target-mRNA. In den Figuren wird gezeigt:
**Fig. 1****:** Die Struktur eines natürlichen Editierungs-Motivs des GluR2-Transkriptes mit der gebundenen ADAR2, die eine Deaminase-Domäne und zwei dsRNA-Bindedomänen (dsRBD1 und dsRBD2) aufweist.
**Fig. 2****:** Die allgemeine Struktur der erfindungsgemäßen guide-RNA. Die Buchstaben A bis I markieren die einzelnen Segmente der guide-RNA;
**Fig. 3****:** Die Struktur der bevorzugten Ausführung der erfindungsgemäßen guide-RNA;
**Fig. 4****:** Die Struktur der konstruierten guide-RNA für die zielgerichtete Editierung von W58X-Stop-Codon im GFP-Gen als Ausführungsbeispiel nach der SEQ ID NO 1;
**Fig. 5****:** Die Struktur der konstruierten guide-RNA für die zielgerichtete Editierung von W58X-Stop-Codon im GFP-Gen als Ausführungsbeispiel nach der SEQ ID NO 3.
**Fig. 6****:** Die Struktur der konstruierten guide-RNA für die zielgerichtete Editierung der R407Q Mutation im "CAG" Codon im PINK1-Gen als Ausführungsbeispiel;
**Fig. 7****:** Die Struktur der konstruierten guide-RNA für die zielgerichtete Editierung der W437X Mutation im "UAG" Codon im PINK1-Gen als Ausführungsbeispiel;
**Fig. 8****:** Die Struktur der konstruierten guide-RNA für die zielgerichtete Editierung der W417X Mutation im "UAG" Codon im Luciferase-Gen als Ausführungsbeispiel;
**Fig. 9****:** Die Struktur der konstruierten guide-RNA für die zielgerichtete Editierung einer krankheitsrelevanten Mutation R521H im "CAC" Codon im FUS-Gen als Ausführungsbeispiel.

### Ausführungsbeispiele

Für die Transfektion der erfindungsgemäßen guide-RNA wurden HEK 293T Zellen verwendet. Für jedes Experiment wurden in einem 24-well-Plattenformat 1,75^{∗}10^5 Zellen am Tag vor der Transfektion ausgelegt. Es wurden Plasmide für die Versuche verwendet, die für humane Zelllinien spezifisch sind. Lipofectamine™ 2000 (Invitrogen) wurde als Transfektionsreagenz eingesetzt.

### Zielgerichtete Editierung von W58X-Stop-Codon im GFP-Gen

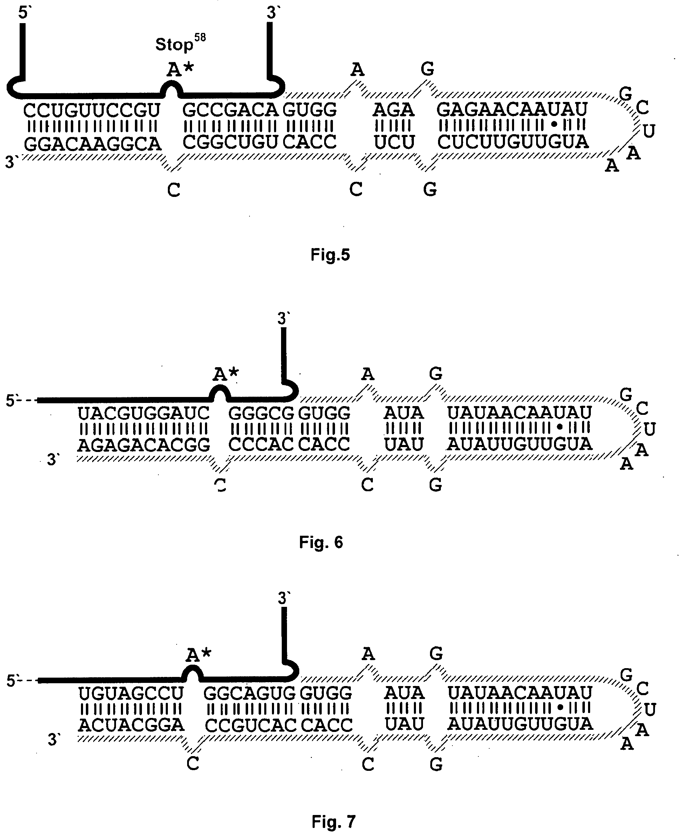

Bei dem W58X handelt es sich um eine Mutation im Codon "TGG", die zum Stop-Codon "TAG" führt. Die für dieses Ausführungsbeispiel konstruierten guide-RNAs sind in **Fig. 4** und **Fig. 5** abgebildet.
a) Standard HEK293T-Zellen wie oben beschrieben
b) HEK293T-Zellen mit induzierbarer ADAR2 Protein Expression (genomisch integriertes ADAR2). Diese integrierte Zelllinie, bei der ADAR2 induziert wird, hat eine ca. 20-fach geringere Expression des Proteins als die Zellen in vorhergehenden Beispielen, die ADAR2 nach einer Transfektion mit je 300 ng Plasmid exprimieren. Die intrazellulären Konzentrationen des ADAR2-Proteins entsprechen somit in diesem Beispiel mehr denen, die in einer natürlichen Zelle vorzufinden sind.
a) Die Zellen wurden mit folgenden Plasmiden co-transfiziert:
   - 300 ng Plasmid, das für W58X kodiert. Das Plasmid ist ein pcDNA3.1 Vektor, der einen CMV-Promotor für die Genexpression von W58X enthält;
   - 300 ng Plasmid, das für hADAR2 kodiert. Das Plasmid ist ein pcDNA3.1 Vektor, der einen CMV-Promotor für die Genexpression von hADAR2 enthält;
   - 1600 ng Plasmid, das für die guide-RNA kodiert. Das Plasmid ist ein 2.1-U6 hygro Vektor, der einen U6 Promotor für die Transkription der guide-RNA enthält.
b) Die Zellen wurden mit folgendem Plasmid co-transfiziert und mit Doxycyclin die hADAR2Expression induziert:
   - 1300 ng Plasmid, das für die guide-RNA kodiert.

Die Beurteilung, ob die Editierung der W58X GFP mRNA stattgefunden hatte, erfolgte sowohl mikroskopisch als auch anhand von RNA-Isolierung.

Im Mikroskop wurde die erfolgreiche Editierung anhand von fluoreszierendem GFP-Signal innerhalb der Zellen gezeigt. Ein positives Editierungs-Ergebnis ist zu sehen, wenn das W58X GFP editiert und damit korrigiert wird. Dadurch ist eine erfolgreiche Editierung im Mikroskop mit fluoreszierenden Zellen gleichzusetzen.

Es wurden in verschiedenen unabhängigen Experimenten die mit den oben genannten Plasmiden transfizierten Zellen im Fluoreszenzmikroskop analysiert und es konnten fluoreszierende Zellen beobachtet werden, was auf eine erfolgreiche Editierung hindeutet. Zusätzlich wurde eine Positivkontrolle analysiert, bei der anstelle des Plasmids für W58X GFP das Plasmid für das korrekte Wildtyp-GFP transfiziert wurde. Wie erwartet, konnten bei der Positivkontrolle im Mikroskop fluoreszierende Zellen beobachtet werden.

Als Negativkontrolle wurden dieselben Experimente durchgeführt, wobei entweder das Plasmid, das für die guide-RNA kodiert, oder das Plasmid, das für das ADAR2 Enzym kodiert, weggelassen wurde. Im Fluoreszenzmikroskop war dabei kein GFP, d.h. keine positive Editierung nachweisbar. Auch in der Sequenzierung dieser RNA-Isolationsproben war keine Editierung nachweisbar, was auf die Spezifität dieses Verfahrens hindeutet.

Bei der Analyse der isolierten RNA erfolgte zunächst eine reverse Transkription zur cDNA, die im Anschluss durch PCR (Polymerase-Kettenreaktion) vervielfältigt wurde, um dann sequenziert zu werden. Das Verhältnis von Adenin zu Guanin in den Sequenzier-Spuren an der Zielstelle gab darüber Auskunft, wie stark die Editierung erfolgte. Im Vergleich zu Negativkontrollen war das Verhältnis von Adenin zu Guanin im Experiment in den mit den oben genannten Plasmiden transfizierten Zellen um einiges höher, was auf eine erfolgreiche Editierung hindeutet. Die Durchführung von Versuchsteil a) lieferte bei der Verwendung des Plasmides, das für die guide-RNA mit der SEQ ID Nr. 1 kodiert, eine Editingausbeute von 58% und mit der guide-RNA mit der SEQ ID Nr. 3 eine Editingausbeute von 38%. Bei der Versuchsdurchführung von Teil b) lieferte die guide-RNA mit der SEQ ID Nr. 1 eine Editingausbeute von 42% und die guide-RNA mit der SEQ ID Nr. 3 eine Editingausbeute von 58%.

### Zielgerichtete Editierunq einer krankheitsrelevanten Mutation im PINK1-Gen

PINK1-Gen steht im Zusammenhang mit der Parkinson Krankheit. In diesem Ausführungsbeispiel wurde die sog. R407Q Mutation im "CAG" Codon editiert.

Die für dieses Ausführungsbeispiel konstruierte guide-RNA ist in Fig. 6 abgebildet.

Die Zellen wurden mit folgenden Plasmiden co-transfiziert:
- 300 ng Plasmid, das für PINK1 R407Q kodiert. Das Plasmid ist ein pcDNA3.1 Vektor, mit einem CMV-Promotor für die Genexpression;
- 300 ng Plasmid, das für hADAR2 kodiert;
- 1600 ng Plasmid, das für die guide-RNA kodiert.

Die Analyse der Editierung erfolgte mittels RNA-Isolierung. Die Editierung in den mit allen o.g. Plasmiden transfizierten Zellen erreichte 35-40% an der Zielstelle. Im anderen Ausführungsbeispiel wurde die W437X Mutation im "TAG" Codon editiert. Dadurch konnte ein krankheitsrelevanter Phänotyp (Verlust der Mitophagie) repariert werden.

Die für dieses Ausführungsbeispiel konstruierte guide-RNA ist in Fig 7 abgebildet.

Die Zellen wurden mit folgenden Plasmiden co-transfiziert:
- 300 ng Plasmid, das für PINK1 W437X kodiert. Das Plasmid ist ein pcDNA3.1 Vektor, mit einem CMV-Promotor für die Genexpression;
- 300 ng Plasmid, das für hADAR2 kodiert;
- 1600 ng Plasmid, das für die guide-RNA kodiert.

Die Analyse der Editierung erfolgte mittels RNA-Isolierung. Die Editierung in den mit allen o.g. Plasmiden transfizierten Zellen erreichte 30% an der Zielstelle. Zusätzlich wurde ein PINK1 Funktionalitätsassay in HeLa Zellen durchgeführt, bei dem der loss-of-function Phänotyp nur dann wiederhergestellt werden konnte (mikroskopische Analyse), wenn hADAR2 und die guide-RNA transfiziert wurden. Die Wiederherstellung der Mitophagie in HeLa Zellen konnte im Mikroskop nachgewiesen werden.

### Zielgerichtete Editierung einer Mutation im Luciferase-Gen

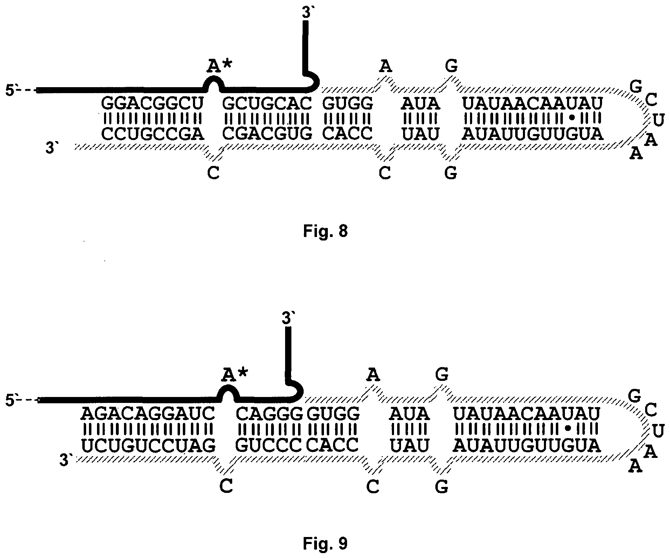

In diesem Ausführungsbeispiel wurde die sog. W417X-Mutation im "TAG" Codon editiert. Die für dieses Ausführungsbeispiel konstruierte guide-RNA ist in Fig. 8 abgebildet.

Die Zellen wurden mit folgenden Plasmiden co-transfiziert:
- 300 ng Plasmid, das für W417X Luciferase kodiert. Das Plasmid ist ein pcDNA3.1 Vektor, mit einem CMV-Promotor für die Genexpression;
- 300 ng Plasmid, das für hADAR2 kodiert;
- 1600 ng Plasmid, das für die guide-RNA kodiert

Die Analyse der Editierung der W417X Luciferase mRNA wurde mittels RNA-Isolierung durchgeführt. Die Editierung in den mit allen o.g. Plasmiden transfizierten Zellen erreichte 48% an der Zielstelle. In der Negativkontrolle wurde das Plasmid mit der spezifischen guide-RNA bei der Transfektion weggelassen. Bei der Negativkontrolle erfolgte keine Editierung, was auf die Ausschließlichkeit der guide-RNA bei der Editierung hindeutet.

### Zielgerichtete Editierung einer krankheitsrelevanten Mutation im FUS-Gen

Dieses Gen steht im Zusammenhang von ALS (Amyotrophic lateral sclerosis), einer tödlichen neuro-degenerativen Funktionsstörung. Die Mutation dieses Gens wird als R521H benannt und beinhaltet ein ,CAC' Codon, während das funktionale FUS ein ,CGC' Codon trägt.

Die für dieses Ausführungsbeispiel konstruierte guide-RNA ist in Fig. 9 abgebildet.

Die Editierung dieses Gens wurde im PCR-Reaktionsgefäß durchgeführt. Hierzu wurden 350nM gereinigtes ADAR2 Protein, 125 nM spezifischen 16 nt langen guide-RNA und 25 nM der mutierten R521H FUS mRNA eingesetzt. Das Sequenzier-Ergebnis zeigt, dass 51% der Adenosine erfolgreichzu Inosin editiert wurden.

### Zielgerichtete Editierungen von endogenen Transkripten durch Transfektion der guide-RNA

Es wurden sechs verschiedene Gene editiert (β-actin, GAPDH, GPI, GUSB, VCP, RAB7A), die unterschiedlich stark endogen expremiert werden. Hierbei handelt es sich nicht um krankheitsrelevante Mutationen, die adressiert wurden, sondern um eine Darstellung der Erreichbarkeit natürlich expremierter mRNAs. Für die Transfektion wurden folgende Zellen verwendet:
a) standard HEK293T-Zellen(wie oben) und
b) HEK-Zellenmit induzierbarer ADAR2 Protein Expression (genomisch integriertes ADAR2). Diese integrierte Zelllinie, bei der ADAR2 induziert wird, hat eine ca. 20-fach geringere Expression des Proteins als die Zellen in vorhergehenden Beispielen, die ADAR2 nach einer Transfektion mit je 300 ng Plasmid exprimieren. Die intrazellulären Konzentrationen des ADAR2-Proteins entsprechen somit in diesem Beispiel mehr denen, die in einer natürlichen Zelle vorzufinden sind.

Die guide-RNA Strukturen, des flexiblen Teils H + I für die jeweiligen Zielgene sind in Tabelle 2 angegeben.

**Tabelle 2: Liste der guideRNAs, die für die Editierung endogener Zielgene verwendet wurden.**

| **Name der guideRNA** | **Sequenz von H+I 5'→3'** |
|---|---|
| TAG#1 β-Actin | ACGCAACCAAGUCAUA |
| TAG#3 β-Actin | GCAAUGCCAUCACCUC |
| TAG#1 GAPDH | AGGGGUCCACAUGGCA |
| TAG#2 GAPDH | GGCUCCCCAGGCCCCU |
| TAG#1 GPI | UGCCGUCCACCAGGAU |
| TAG#1 GusB | CAGAUUCCAGGUGGGA |
| TAG#2 GusB | UCCCUGCCAGAAUAGA |
| TAG#1 VCP | CUCCGCCCACCAAAUG |
| TAG#2 VCP | CCCAAACCACAACAGA |
| TAG#3 VCP | ACCCACCCACCCAGGU |
| TAG#1 RAB7A | CUGCCGCCAGCUGGAU |
| TAG#2 RAB7A | AGGGAACCAGACAGUU |

a) Die Zellen wurden mit folgenden Plasmiden co-transfiziert:
   - 300 ng Plasmid, das für hADAR2 kodiert;
   - 1300 ng Plasmid, das für die guide-RNA kodiert.

   Die Analyse der Editierung erfolgte mittels RNA-Isolierung. Das Verhältnis von Adenin zu Guanin bei den mit diesen Plasmiden transfizierten Zellen war um einiges höher als in den Negativkontrollen, was auf eine erfolgreiche Editierung hindeutet.
b) Die Zellen wurden mit folgendem Plasmid co-transfiziert und mit Doxycyclin die hADAR2 Expression induziert:
   - 1300 ng Plasmid, das für die guide-RNA kodiert.

Die Analyse der Editierung erfolgte mittels RNA-Isolierung. Das Verhältnis von Adenin zu Guanin bei den mit diesen Plasmiden transfizierten Zellen war um einiges höher als in den Negativkontrollen, was auf eine erfolgreiche Editierung hindeutet.

| Gen | Versuchsteil a) | Versuchsteil b) |
|---|---|---|
| **β-Actin** | | |
| TAG#1 | 26% | 16% |
| TAG#3 | 24% | 14% |
| **GAPDH** | | |
| TAG#1 | 19% | 10% |
| TAG#2 | 20% | 11% |
| **GPI** | | |
| TAG#1 | 16% | 12% |
| **GUSB** | | |
| TAG#1 | 10% | 10% |
| TAG#2 | 23% | 18% |
| **VCP** | | |
| TAG#1 | 23% | 23% |
| TAG#2 | 16% | 15% |
| TAG#3 | 12% | 13% |
| **RAB7A** | | |
| TAG#1 | 31% | 38% |
| TAG#2 | 28% | 35% |

### Referenzen

1) T. Stafforst, M. F. Schneider; Ein RNA-Deaminase-Konjugat ermöglicht die selektive Reparatur von Punktmutationen. Angew. Chem. 2012, 124, 11329-32.
2) Marius F. Schneider, Jacqueline Wettengel, Patrick C. Hoffmann and Thorsten Stafforst; Optimal guideRNAs for re-directing deaminase activity of hADAR1 and hADAR2 in trans. Nucleic Acids Research, 2014, Vol. 42, No. 10 e87
3) Montiel-Gonzales M.F., Vallecillo-Viejo, I., Yudowski, G.A. and Rosenthal, J.J. (2013) Correction of mutations within the cystic fibrosis transmembrane conductance regulator by site-directed RNA editing. Proceedings of the National Academy of Sciences of the United States of America, 110, 18285-18290
4) Vogel, P. and Stafforst, T. (2014) Site-directed RNA editing with antagomir deaminases - a tool to study protein and RNA function. ChemMedChem, 9, 2021-2025.
5) Nishikura, K. (2010) Functions and regulation of RNA editing by ADAR deaminases. Annual review of biochemistry, 79, 321.
6) Vogel, P., Schneider, M.F., Wettengel, J. and Stafforst, T. (2014) Improving site-directed RNA editing in vitro and in cell culture by chemical modification of the guideRNA. Angewandte Chemie, 53, 6267-6271.
7) Bass, B.L. (2002) RNA editing by adenosine deaminases that act on RNA. Annual review of biochemistry, 71, 817.
8) Stefl, R., Oberstrass, F.C., Hood, J.L., Jourdan, M., Zimmermann, M., Skrisovska, L., Maris, C., Peng, L., Hofr, C. and Emeson, R.B. (2010) The solution structure of the ADAR2 dsRBM-RNA complex reveals a sequencespecific readout of the minor groove. Cell, 143, 225-237.
9) Hanswillemenke, A., Kuzdere, T., Vogel, P., Jékely, G. and Stafforst, T. (2015) Site-Directed RNA Editing in Vivo can be Triggered by the Light-Driven Assembly of an Artificial Riboprotein. Journal of the American Chemical Society, 137, 15875-15881.

### Sequenzprotokoll

<110> Eberhard Karls Universität Tübingen
<120> Verfahren und Substanzen zur gerichteten RNA-Editierung
<130>
<150> DE 10 2015 012 522
<151> 26.09.2015
<160> 7
<210> SEQ ID NO: 1
   <211> 44
   <212> RNA
   <213> künstliche Sequenz
<400> 1
   guggauagua uaacaauaug cumaauguug uuauaguauc ccac 44
<110> Eberhard Karls Universität Tübingen
<120> Verfahren und Substanzen zur gerichteten RNA-Editierung
<130>
<150> DE 10 2015 012 522
   <151> 26.09.2015
<160> 7
<210> SEQ ID NO: 2
   <211> 45
   <212> RNA
   <213> künstliche Sequenz
<400> 1
   guggasssgs asascaauau gcumaauguu gsususgsss cccac 45
<110> Eberhard Karls Universität Tübingen
<120> Verfahren und Substanzen zur gerichteten RNA-Editierung
<130>
<150> DE 10 2015 012 522
<151> 26.09.2015
<160> 7
<210> SEQ ID NO: 3
   <211> 45
   <212> RNA
   <213> künstliche Sequenz
<400> 1
   guggaasags asaacaauau gcumaauguu guususgusu cccac 45
<110> Eberhard Karls Universität Tübingen
<120> Verfahren und Substanzen zur gerichteten RNA-Editierung
<130>
<150> DE 10 2015 012 522
   <151> 26.09.2015
<160> 7
<210> SEQ ID NO: 4
   <211> 44
   <212> RNA
   <213> künstliche Sequenz
<400> 1
   guggasasua uaacaauaug cumaauguug uuauagyayc ccac 44
<110> Eberhard Karls Universität Tübingen
<120> Verfahren und Substanzen zur gerichteten RNA-Editierung
<130>
<150> DE 10 2015 012 522
<151> 26.09.2015
<160> 7
<210> SEQ ID NO: 5
   <211> 45
   <212> RNA
   <213> künstliche Sequenz
<400> 1
   guggasssgs sssssssuau gcumaaugss ssssssgsss cccac 45
<110> Eberhard Karls Universität Tübingen
<120> Verfahren und Substanzen zur gerichteten RNA-Editierung
<130>
<150> DE 10 2015 012 522
<151> 26.09.2015
<160> 7
<210> SEQ ID NO: 6
   <211> 45
   <212> RNA
   <213> künstliche Sequenz
<400> 1
   guggaassgs asascaauau gcumaauguu gsususgssu cccac 45
<110> Eberhard Karls Universität Tübingen
<120> Verfahren und Substanzen zur gerichteten RNA-Editierung
<130>
<150> DE 10 2015 012 522
<151> 26.09.2015
<160> 7
<210> SEQ ID NO: 7
   <211> 46
   <212> RNA
   <213> künstliche Sequenz
<400> 1
   guggsauagu auaacaauau gcumaauguu guuauagüau scccac 46

Die einzelnen Buchstaben stehen für: a für Adenin, g für Guanin, c für Cytosin, u für Uracil, s für ein Guanin oder ein Cytosin, und m für ein Adenin oder Cytosin.

## Patentansprüche

1. Guide-RNA zur gerichteten RNA-Editierung mit mindestens folgenden aneinandergekoppelten Nukleotid-Segmenten, vom 5'-Ende aufgelistet:
- Segment A: Nukleotid-Sequenz mit einer Länge von drei bis fünf Basen, wobei in der Position 1 immer ein Guanosin und in der Position 2 immer ein Uridin steht;
- Segment B: Nukleotid-Sequenz mit einer Länge von drei bis fünf Basen, wobei in der Position 1 immer ein Adenosin steht;
- Segment C: Nukleotid-Sequenz mit einer Länge von acht bis zehn Basen, wobei in der Position 1 immer ein Guanosin steht;
- Segment D: Nukleotid-Sequenz UAUGCUAAAUG oder UAUGCUCAAUG;
- Segment E: Nukleotid-Sequenz mit einer Länge von acht bis zehn Basen, wobei in der letzten Position immer ein Guanosin steht;
- Segment F: Nukleotid-Sequenz mit einer Länge von drei bis fünf Basen, wobei in der letzten Position immer ein Cytosin steht;
- Segment G: Nukleotid-Sequenz mit einer Länge von drei bis fünf Basen, wobei in der vorletzten Position immer ein Adenosin und in der letzten Position ein Cytosin steht;
- Segment H: Nukleotid-Sequenz mit einer Länge von fünf bis neun Basen, wobei in der letzten Position immer ein Cytosin oder ein Uridin steht;
- Segment I: Nukleotid-Sequenz mit einer Länge von acht bis zwanzig Basen,
wobei einzelne Nukleotide der Segmente A und G, B und F bzw. C und E sich jeweils zu einer Doppelhelix paaren und die Nukleotide des Segments D eine Haarnadelstruktur bilden, und
wobei Segmente H und I so konstruiert sind, dass sie mit der zu editierenden mRNA paaren.

2. Guide-RNA nach Anspruch 1, wobei Segment A die Länge von vier Nukleotiden, insbesondere die Nukleotid-Sequenz GUGG aufweist.

3. Guide-RNA nach mindestens einem der vorhergehenden Ansprüche, wobei Segment B die Länge von vier Nukleotiden, insbesondere die Nukleotid-Sequenz AAUA aufweist.

4. Guide-RNA nach mindestens einem der vorhergehenden Ansprüche, wobei Segment C die Länge von neun Nukleotiden, insbesondere die Nukleotid-Sequenz GUAUAACAA aufweist.

5. Guide-RNA nach mindestens einem der vorhergehenden Ansprüche, wobei Segment E die Länge von neun Nukleotiden, insbesondere die Nukleotid-Sequenz UUGUUAUAG aufweist.

6. Guide-RNA nach mindestens einem der vorhergehenden Ansprüche, wobei Segment F die Länge von vier Nukleotiden, insbesondere die Nukleotid-Sequenz UAUC aufweist.

7. Guide-RNA nach mindestens einem der vorhergehenden Ansprüche, wobei Segment G die Länge von vier Nukleotiden, insbesondere die Nukleotid-Sequenz CCAC aufweist.

8. Guide-RNA nach mindestens einem der vorhergehenden Ansprüche, wobei Segmente H und I so konstruiert sind, dass sie die zu editierende Base in ein A:C Fehlpaar platzieren.

9. Guide-RNA nach mindestens einem der vorhergehenden Ansprüche, wobei am 3'-Ende eine Haarnadelstruktur, insbesondere ein BoxB-Motiv, angehängt ist.

10. Guide-RNA nach mindestens einem der vorhergehenden Ansprüche zur Verwendung als Arzneimittel, wobei die Guide-RNA in die Zellen transfiziert wird, in denen die RNA-Editierung vorgenommen werden soll.

11. Guide-RNA zur Verwendung nach Anspruch 10, wobei die Transfektion mittels eines Plasmids erfolgt, welches für die guide-RNA sowie mindestens einen U6-Promotor für die Transkription der guide-RNA kodiert.

12. Guide-RNA zur Verwendung nach einem der Ansprüche 10 oder 11 zur gezielten Veränderung genetischer Information auf der RNA-Ebene, bevorzugt für Reparaturen einzelner Punktmutationen.

13. In-vitro-Verfahren zur gerichteten RNA-Editierung, bei dem die Guide-RNA nach mindestens einem der Ansprüche 1 bis 9 wie in einem der Ansprüche 10 oder 11 definiert in die Zellen transfiziert wird.

14. Verwendung der Guide-RNA nach einem der Ansprüche 1 bis 9 in vitro zur gezielten Veränderung genetischer Information auf der RNA-Ebene, bevorzugt für Reparaturen einzelner Punktmutationen.

## Claims

1. Guide RNA for targeted RNA editing having at least the following nucleotide segments coupled to one another, listed from the 5' terminus:
- Segment A: Nucleotide sequence having a length of three to five bases, wherein a guanosine is always in position 1 and a uridine is always in position 2;
- Segment B: Nucleotide sequence having a length of three to five bases, wherein an adenosine is always in position 1;
- Segment C: Nucleotide sequence having a length of eight to ten bases, wherein a guanosine is always in position 1;
- Segment D: Nucleotide sequence UAUGCUAAAUG or UAUGCUCAAUG;
- Segment E: Nucleotide sequence having a length of eight to ten bases, wherein a guanosine is always in the ultimate position;
- Segment F: Nucleotide sequence having a length of three to five bases, wherein a cytosine is always in the ultimate position;
- Segment G: Nucleotide sequence having a length of three to five bases, wherein an adenosine is always in the next-to-the-ultimate position and a cytosine is in the ultimate position;
- Segment H: Nucleotide sequence having a length of five to nine bases, wherein a cytosine or a uridine is always in the ultimate position;
- Segment I: Nucleotide sequence having a length of eight to twenty bases,
wherein individual nucleotides of the segments A and G, B and F, or C and E pair to form a double helix and the nucleotides of segment D form a hairpin structure and
wherein segments H and I are designed such that they pair with the mRNA to be edited.

2. The guide RNA according to claim 1, wherein segment A has the length of four nucleotides, in particular has the GUGG nucleotide sequence.

3. The guide RNA according to at least one of the preceding claims, wherein segment B has the length of four nucleotides, in particular has the AAUA nucleotide sequence.

4. The guide RNA according to at least one of the preceding claims, wherein segment C has the length of nine nucleotides, in particular has the GUAUAACAA nucleotide sequence.

5. The guide RNA according to at least one of the preceding claims, wherein segment E has the length of nine nucleotides, in particular has the UUGUUAUAG nucleotide sequence.

6. The guide RNA according to at least one of the preceding claims, wherein segment F has the length of four nucleotides, in particular has the UAUC nucleotide sequence.

7. The guide RNA according to at least one of the preceding claims, wherein segment G has the length of four nucleotides, in particular has the CCAC nucleotide sequence.

8. The guide RNA according to at least one of the preceding claims, wherein segments H and I are designed such that they place the base to be edited in an A:C mismatch pair.

9. The guide RNA according to at least one of the preceding claims, wherein a hairpin structure, especially a BoxB motif, is appended on the 3' terminus.

10. Guide RNA according to at least one of the preceding claims for use as a medicament, wherein the guide RNA is transfected into cells, in which the RNA editing is to be carried out.

11. Guide RNA for use according to claim 10, wherein the transfection occurs by means of a plasmid that codes for the guide RNA and at least one U6 promoter for the transcription for the guide RNA.

12. Guide RNA for use according to one of claims 10 or 11 for targeted alteration of genetic information on the RNA level, preferably for repair of individual point mutations.

13. In-vitro method for directed RNA editing, wherein the guide RNA according to at least one of the claims 1 to 9 is transfected into the cells as defined in claim 10 or 11.

14. Use of the guide RNA according to any of the claims 1 to 9 in-vitro for targeted alteration of genetic information on the RNA level, preferably for the repair of individual point mutations.

## Revendications

1. ARN guide pour l'édition ciblée d'ARN, présentant au moins les segments nucléotidiques suivants, couplés les uns aux autres, énumérés à partir de l'extrémité 5' :
- segment A : séquence nucléotidique d'une longueur de trois à cinq bases, la position 1 étant toujours occupée par une guanosine et la position 2 étant toujours occupée par une uridine ;
- segment B : séquence nucléotidique d'une longueur de trois à cinq bases, la position 1 étant toujours occupée par une adénosine ;
- segment C : séquence nucléotidique d'une longueur de huit à dix bases, la position 1 étant toujours occupée par une guanosine ;
- segment D : séquence nucléotidique UAUGCUAAAUG ou UAUGCUCAAUG;
- segment E : séquence nucléotidique d'une longueur de huit à dix bases, la dernière position étant toujours occupée par une guanosine ;
- segment F : séquence nucléotidique d'une longueur de trois à cinq bases, la dernière position étant toujours occupée par une cytosine ;
- segment G : séquence nucléotidique d'une longueur de trois à cinq bases, l'avant-dernière dernière position étant toujours occupée par une adénosine et la dernière position étant toujours occupée par une cytosine ;
- segment H : séquence nucléotidique d'une longueur de cinq à neuf bases, la dernière position étant toujours occupée par une cytosine ou une uridine ;
- segment I : séquence nucléotidique d'une longueur de huit à vingt bases,
certains nucléotides des segments A et G, B et F ou C et E s'appariant toujours en une double hélice et les nucléotides du segment D formant une structure en épingle à cheveux et les segments H et I étant construits de manière telle qu'ils s'apparient à l'ARNm à éditer.

2. ARN guide selon la revendication 1, le segment A présentant la longueur de quatre nucléotides, en particulier la séquence nucléotidique GUGG.

3. ARN guide selon au moins l'une quelconque des revendications précédentes, le segment B présentant la longueur de quatre nucléotides, en particulier la séquence nucléotidique AAUA.

4. ARN guide selon au moins l'une quelconque des revendications précédentes, le segment C présentant la longueur de neuf nucléotides, en particulier la séquence nucléotidique GUAUAACAA.

5. ARN guide selon au moins l'une quelconque des revendications précédentes, le segment E présentant la longueur de neuf nucléotides, en particulier la séquence nucléotidique UUGUUAUAG.

6. ARN guide selon au moins l'une quelconque des revendications précédentes, le segment F présentant la longueur de quatre nucléotides, en particulier la séquence nucléotidique UAUC.

7. ARN guide selon au moins l'une quelconque des revendications précédentes, le segment G présentant la longueur de quatre nucléotides, en particulier la séquence nucléotidique CCAC.

8. ARN guide selon au moins l'une quelconque des revendications précédentes, les segments H et I étant construits de manière telle qu'ils placent la base à éditer dans une paire erronée A:C.

9. ARN guide selon au moins l'une quelconque des revendications précédentes, une structure en épingle à cheveux, en particulier un motif BoxB, étant attachée à l'extrémité 3'.

10. ARN guide selon au moins l'une quelconque des revendications précédentes, pour une utilisation en tant que médicament, l'ARN guide étant transfecté dans les cellules dans lesquelles l'édition d'ARN doit être effectuée.

11. ARN guide pour une utilisation selon la revendication 10, la transfection ayant lieu au moyen d'un plasmide qui code pour l'ARN guide ainsi que pour au moins un promoteur U6 pour la transcription de l'ARN guide.

12. ARN guide pour une utilisation selon l'une quelconque des revendications 10 ou 11 pour la modification ciblée d'informations génétiques sur le plan de l'ARN, de préférence pour des réparations de certaines mutations ponctuelles.

13. Procédé in vitro pour l'édition ciblée d'ARN, dans lequel l'ARN guide selon au moins l'une quelconque des revendications 1 à 9 est transfecté comme défini dans l'une quelconque des revendications 10 ou 11 dans les cellules.

14. Utilisation de l'ARN guide selon l'une quelconque des revendications 1 à 9 in vitro pour la modification ciblée d'informations génétiques sur le plan de l'ARN, de préférence pour des réparations de certaines mutations ponctuelles.
